# FASCICULE DE BREVET EUROPEEN

(11) **EP 4 034 024 B1**
(45) Date de publication et mention de la délivrance du brevet: **25.10.2023**
(21) Numéro de dépôt: 20772313.1
(22) Date de dépôt: 22.09.2020
(51) Int. Cl.: A61B 34/30, B25J 9/10, B25J 18/00, B25J 9/16

(54) **DISPOSITIF D'AIDE A LA CHIRURGIE**
SURGERY ASSISTANCE DEVICE
CHIRURGISCHES HILFSGERÄT

(30) Priorité: 24.09.2019 FR 1910523
(43) Date de publication de la demande: 03.08.2022
(73) Titulaire: CENTRE NATIONAL DE LA RECHERCHE SCIENTIFIQUE (CNRS), 75016 Paris (FR); CHU de Nantes, 44000 Nantes (FR)
(72) Inventeur: CHABLAT, Damien, 44240 La Chapelle-sur-Erdre (FR); MICHEL, Guillaume, 44230 Saint-Sébastien-sur-Loire (FR); BORDURE, Philippe, 44100 Nantes (FR)
(74) Mandataire: Cabinet Le Guen Maillet
(86) Numéro de dépôt international: PCT/EP2020/076353
(87) Numéro de publication internationale: WO 2021/058448

(56) Documents cités:
- DE-T2- 69 310 085
- FR-A1- 2 845 889
- US-A- 5 817 084
- US-A1- 2019 053 863

## Description

### DOMAINE TECHNIQUE

La présente invention concerne le domaine de l'assistance chirurgicale. La présente invention concerne plus précisément le domaine des robots chirurgicaux destinés à assister un chirurgien lors d'une intervention chirurgicale.

### ETAT DE LA TECHNIQUE ANTERIEURE

Lors d'une opération de chirurgie, un chirurgien utilise généralement ses deux mains pour tenir, dans l'une, un instrument chirurgical, et dans l'autre, un outil d'aspiration. Dans certains cas, les opérations de chirurgie sont réalisées dans des zones d'intervention chirurgicale difficiles d'accès qui ne sont pas entièrement observables depuis l'extérieur avec un microscope ou une loupe binoculaire. C'est le cas par exemple en chirurgie otologique car l'oreille comporte un conduit auditif étroit derrière lequel se trouve une cavité plus large. Afin de visualiser l'intégralité de la zone d'intervention chirurgicale, le chirurgien doit alors recourir à l'utilisation d'un endoscope. En contrepartie, le chirurgien doit lâcher l'outil d'aspiration, ce qui est également problématique. En effet, lorsque l'opération génère des saignements importants, comme c'est souvent le cas en chirurgie otologique, l'absence d'outil d'aspiration dégrade la visibilité et implique que l'endoscope doit être retiré fréquemment pour être nettoyé. La répétition d'insertions et extractions de l'endoscope au cours de l'opération requiert de la précision et est chronophage pour le chirurgien. Par ailleurs, certains incidents pouvant survenir lors d'une opération, comme par exemple le réveil du patient, nécessitent de retirer l'endoscope de manière sécurisée et le plus rapidement possible afin d'éviter de blesser le patient.

Il est souhaitable de pallier ces inconvénients de l'état de la technique. Il est notamment souhaitable de fournir une solution qui permette d'insérer et d'extraire un endoscope dans une zone d'intervention difficile d'accès de manière automatisée, fiable et sécurisée. Il est en particulier souhaitable de fournir une solution qui permette de maintenir et d'orienter un endoscope dans une zone d'intervention tout en limitant l'occupation de l'espace de travail du chirurgien au-dessus du patient. Il est également souhaitable de fournir une solution qui permette une extraction rapide et automatique de l'endoscope en cas de la survenue d'un incident et qui permette en outre de réinsérer l'endoscope avec précision à une position précédente. DE69310085, US2019/053863, US5817084 et FR2845889 décrivent l'art antérieur.

### EXPOSE DE L'INVENTION

Un objet de la présente invention est de proposer un dispositif d'aide à la chirurgie comportant des moyens pour déporter une rotation de premier type et une rotation de deuxième type et comportant en outre un mécanisme de transmission d'une rotation de troisième type et un mécanisme de transformation de la rotation de troisième type en une translation. Ladite translation est autorisée selon un axe d'insertion orienté dans une direction définie par les moyens pour déporter la rotation de premier type et la rotation de deuxième type. Le mécanisme de transmission de la rotation de troisième type est relié à une première extrémité d'un élément élastique, la deuxième extrémité de l'élément élastique est reliée aux moyens pour déporter la rotation de premier type et la rotation de deuxième type. Le mécanisme de transmission de la rotation de troisième type est relié à un moteur rotatif, la rotation du moteur rotatif dans un premier sens provoquant l'abaissement d'un outil porté par le dispositif d'aide à la chirurgie et une première déformation de l'élément élastique, la rotation du moteur rotatif dans un deuxième sens provoquant l'élévation de l'outil et une deuxième déformation de l'élément élastique opposée à la première déformation, l'outil restant dans une position donnée tant qu'un couple exercé par le moteur rotatif est maintenu et, lorsque l'outil est dans une position abaissée et que le moteur rotatif n'exerce aucun couple, l'élément élastique revient à une forme initiale provoquant l'élévation de l'outil. Ainsi, l'outil peut être inséré dans et extrait de la zone d'intervention chirurgicale de manière fiable et automatisée et l'outil peut être extrait de manière sécurisée et rapide en cas d'incident.

Selon un mode de réalisation particulier, la première déformation est une élongation. Ainsi, l'élément élastique génère une traction sur le mécanisme de transmission de la rotation de troisième type qui tend à entraîner ladite rotation de troisième type dans un sens entraînant l'élévation de l'outil et donc son extraction de la zone chirurgicale.

Selon un mode de réalisation particulier, les moyens pour déporter la rotation de premier type et la rotation de deuxième type comportent deux parallélogrammes reliés entre eux et un dispositif d'actionnement de la rotation de premier type et de la rotation de deuxième type. Le dispositif d'actionnement génère un mouvement de rotation de premier type, autour d'un premier axe, d'une tige d'un parallélogramme par rapport à une autre tige dudit parallélogramme, et génère un mouvement de rotation de deuxième type, autour d'un deuxième axe, des deux parallélogrammes par rapport à une base du dispositif d'aide à la chirurgie, le premier axe étant perpendiculaire au deuxième axe, et les mouvements de rotation de premier type et de deuxième type étant transmis par les deux parallélogrammes en un centre de rotation déporté.

Ainsi, l'outil peut être maintenu et orienté de manière automatisée dans la zone d'intervention chirurgicale grâce à la déportation du centre de rotation tout en limitant l'occupation d'un espace de travail du chirurgien, situé au-dessus de la zone d'intervention chirurgicale.

Selon un mode de réalisation particulier, le dispositif d'actionnement comporte deux autres moteurs rotatifs, chacune des rotations de premier et deuxième type étant actionnée par l'un des deux autres moteurs rotatifs.

Ainsi, l'orientation de l'outil est actionnée à distance.

Selon un mode de réalisation particulier, le dispositif d'actionnement comporte deux moteurs linéaires, les rotations de premier et deuxième type étant actionnées par l'action conjointe des deux moteurs linéaires.

Ainsi, l'orientation de l'outil est actionnée à distance.

Selon un mode de réalisation particulier, le mécanisme de transmission de la rotation de troisième type comporte un troisième parallélogramme. Le moteur rotatif génère un mouvement de rotation de troisième type, autour d'un troisième axe, d'un premier élément rotatif par rapport aux moyens pour déporter la rotation de premier type et la rotation de deuxième type, le mouvement de rotation de troisième type étant transmis par le troisième parallélogramme à un deuxième élément rotatif, autour d'un quatrième axe parallèle au troisième axe.

Ainsi, l'outil peut être inséré dans et extrait de la zone d'intervention chirurgicale grâce à un actionnement à distance, ce qui limite l'occupation de l'espace de travail du chirurgien.

Selon un mode de réalisation particulier, le mécanisme de transmission de la rotation de troisième type comporte une courroie. Le moteur rotatif génère un mouvement de rotation de troisième type, autour d'un troisième axe, d'un premier élément rotatif par rapport aux moyens pour déporter la rotation de premier type et la rotation de deuxième type, le mouvement de rotation de troisième type étant transmis par la courroie à un deuxième élément rotatif, autour d'un quatrième axe parallèle au troisième axe.

Ainsi, l'outil peut être inséré dans et extrait de la zone d'intervention chirurgicale grâce à un actionnement à distance, ce qui limite l'occupation de l'espace de travail du chirurgien.

Selon un mode de réalisation particulier, le mécanisme de transformation de la rotation de troisième type en une translation comporte une bielle. La tête de la bielle est reliée au mécanisme de transmission de la rotation de troisième type et le pied de la bielle est relié à l'outil et aux moyens pour déporter la rotation de premier type et la rotation de deuxième type, et lorsque le mouvement de rotation de troisième type est transmis à la tête de la bielle, le pied de la bielle transmet à l'outil un mouvement de translation selon l'axe d'insertion. Ainsi, l'outil est toujours orienté dans la direction de l'axe d'insertion et peut être inséré dans et extrait de la zone d'intervention chirurgicale à tout moment sans risquer d'endommager un espace de passage étroit, tel qu'un conduit auditif.

Selon un mode de réalisation particulier, le mécanisme de transformation de la rotation de troisième type en une translation comporte une roue dentée reliée au mécanisme de transmission de la rotation de troisième type et comporte une crémaillère fixée sur un porte-outil auquel est relié l'outil, la roue dentée étant reliée à la crémaillère par un engrenage de sorte que lorsque la roue dentée entre en mouvement selon la rotation de troisième type, l'outil effectue un mouvement de translation selon l'axe d'insertion.

Ainsi, l'outil est toujours orienté dans la direction de l'axe d'insertion et peut être inséré dans et extrait de la zone d'intervention chirurgicale à tout moment sans risquer d'endommager un espace de passage étroit, tel qu'un conduit auditif.

Selon un mode de réalisation particulier, les moyens pour déporter la rotation de premier type et la rotation de deuxième type sont reliés à une base mobile, la base mobile pouvant être déplacée et fixée sur une table d'opération selon trois axes de translation perpendiculaires entre eux.

Ainsi, le dispositif d'aide à la chirurgie peut être déplacé avec précision sur une table d'opération préalablement à une intervention chirurgicale de sorte que l'outil soit positionné au-dessus de la zone d'intervention chirurgicale.

Selon un mode de réalisation particulier, le dispositif d'aide à la chirurgie comporte en outre un mécanisme de rotation de l'outil autour de l'axe d'insertion.

Ainsi, l'outil peut tourner sur lui-même dans la zone d'intervention chirurgicale et observer une cavité sous différents angles de vue, notamment dans le cas d'un endoscope dont la direction centrale du champ de vue est différente de 0° par rapport à l'axe d'insertion de l'outil.

Selon un mode de réalisation particulier, l'outil est un endoscope pour aider la chirurgie otologique et sinusienne.

Selon un mode de réalisation particulier, le dispositif d'aide à la chirurgie comporte en outre un moyen de commande actionné par un individu et annulant le couple exercé par le moteur rotatif de sorte que si l'outil est dans une position abaissée, l'élément élastique revient à la forme initiale provoquant l'élévation de l'outil.

### BREVE DESCRIPTION DES DESSINS

Les caractéristiques de l'invention mentionnées ci-dessus, ainsi que d'autres, apparaîtront plus clairement à la lecture de la description suivante d'au moins un exemple de réalisation, ladite description étant faite en relation avec les dessins joints, parmi lesquels :
[Fig. 1] illustre schématiquement une vue de côté d'un robot destiné à aider la chirurgie selon la présente invention comportant une base, un bras et un système d'insertion-extraction d'un outil, le système d'insertion-extraction étant dans une position telle que l'outil est abaissé et situé dans une zone d'intervention chirurgicale ;
[Fig. 2] illustre schématiquement une vue de côté du robot dont le bras présente une inclinaison liée à une rotation de premier type, d'axe (Oy) ;
[Fig. 3] illustre schématiquement une vue de derrière du robot ;
[Fig. 4] illustre schématiquement en vue de derrière le robot dont le bras présente une inclinaison liée à une rotation de deuxième type, d'axe (Ox) ;
[Fig. 5] illustre schématiquement le robot en vue de côté, le système d'insertion-extraction étant présenté dans une position relevée avec l'outil situé hors de la zone d'intervention chirurgicale ;
[Fig. 6] illustre un schéma cinématique du mécanisme du robot ; et
[Fig. 7] illustre schématiquement le robot en vue de côté, le système d'insertion-extraction étant présenté dans une position relevée avec l'outil situé hors de la zone d'intervention chirurgicale et comportant une roue dentée associée à une crémaillère.

### EXPOSE DETAILLE DE MODES DE REALISATION

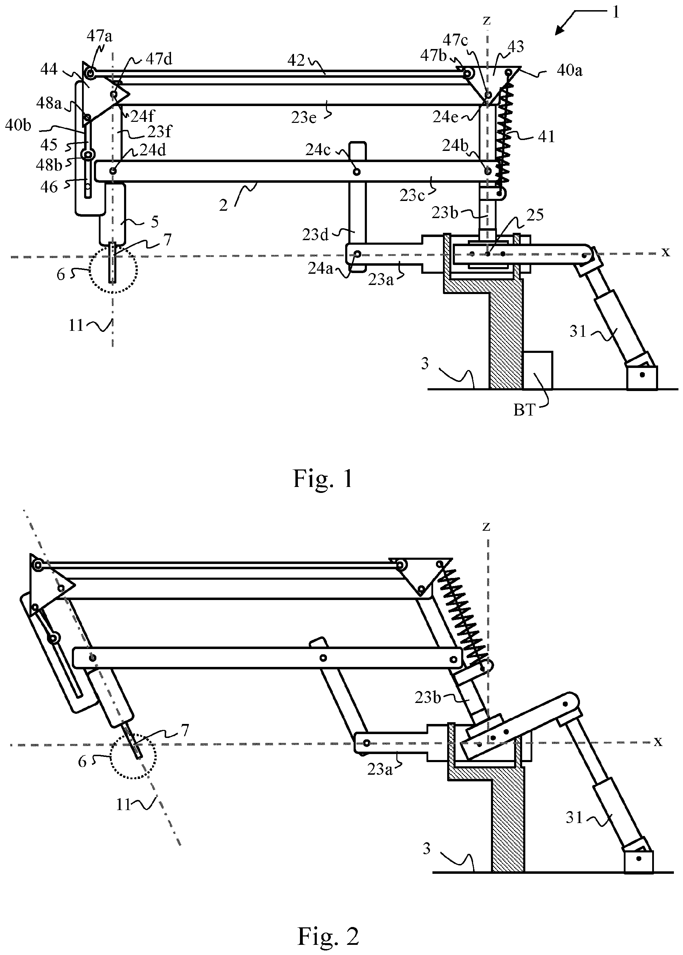

La **Fig. 1** illustre schématiquement une vue de côté d'un robot 1 ou dispositif d'aide à la chirurgie otologique comportant un système d'insertion-extraction d'un outil 5 tel que par exemple un endoscope. Le système d'insertion-extraction est dans une position telle que l'outil 5 est abaissé et situé dans une zone d'intervention chirurgicale 6. Le robot 1 comporte une base 3, un bras 2, le système d'insertion-extraction et l'outil 5. Le robot 1 est schématisé dans un repère (Oxyz), le point O étant situé à l'intersection d'un axe (Ox) et d'un axe (Oz) et un axe (Oy) étant perpendiculaire au plan (Oxz). La zone d'intervention chirurgicale 6 se trouve à proximité de l'intersection entre un axe d'insertion 11 et de l'axe (Ox).

Le bras 2 du robot 1 comporte un double parallélogramme déformable composé d'un premier parallélogramme comportant quatre tiges 23a, 23b, 23c, 23d et relié à un deuxième parallélogramme comportant quatre tiges 23b, 23c, 23e, 23f. La tige arrière 23b verticale et la tige intermédiaire horizontale 23c sont communes aux deux parallélogrammes. Les tiges 23a à 23f sont reliées entre elles par des liaisons pivot 24a à 24f permettant des rotations d'axes parallèles à l'axe (Oy) ainsi qu'une liaison 25 matérialisée par un cardan et autorisant notamment une rotation d'axe (Oy) entre la tige inférieure 23a et la tige arrière 23b. L'axe d'insertion 11 de l'outil 5 est orienté dans la direction de la tige avant 23f, parallèle aux tiges arrière 23b et intermédiaire verticale 23d.

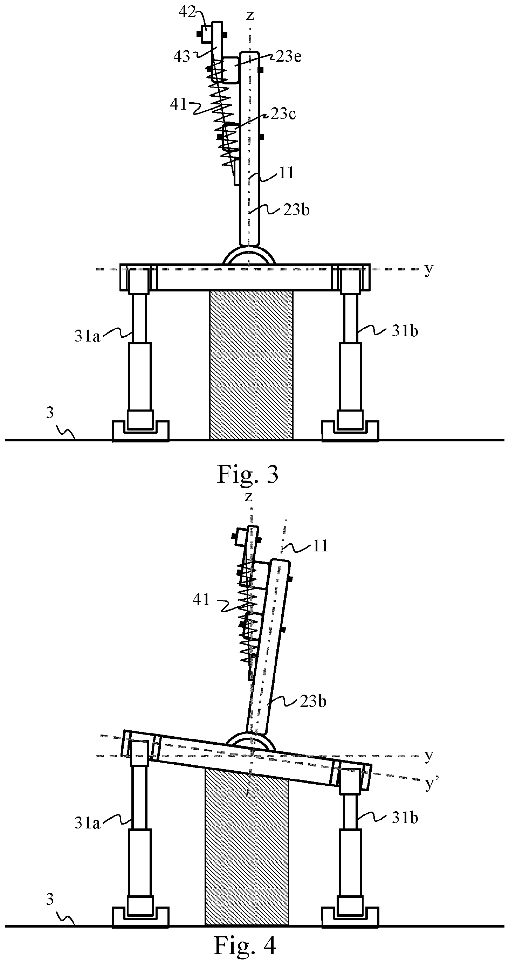

Le bras 2 du robot 1 est relié à la base 3 par un mécanisme d'actionnement permettant d'actionner deux rotations de la tige arrière 23b par rapport la base 3 : une rotation de premier type, d'axe (Oy), et une rotation de deuxième type, d'axe (Ox). Chacune des rotations de premier et deuxième type peut être actionnée indépendamment et les deux rotations de premier et deuxième type peuvent être combinées. Selon un mode de réalisation particulier présenté en Fig. 1, l'actionnement des rotations de premier et deuxième type, d'axe (Oy) et d'axe (Ox), est effectué grâce à l'action conjointe de deux moteurs linéaires 31 parallèles. Les moteurs linéaires 31a et 31b sont également représentés en Figs. 3 et 4. Alternativement, l'actionnement de chacune des rotations de premier type et de deuxième type est effectué grâce à un moteur rotatif. En outre, le bras 2 du robot 1 est relié à la base 3 par une liaison pivot d'axe (Ox) entre la tige inférieure 23a et la base 3, permettant de supprimer des degrés de liberté, et notamment d'empêcher une rotation d'axe (Oy) entre la tige inférieure 23a et la base 3 de sorte que la tige inférieure 23a reste en permanence horizontale, dans le prolongement de l'axe (Ox).

Lorsque la rotation de deuxième type est actionnée par le mécanisme d'actionnement, la tige arrière 23b entraîne l'ensemble du bras 2 du robot 1 dans le mouvement de rotation de deuxième type, en raison de l'absence de degré de liberté selon la rotation d'axe (Ox) entre la tige arrière 23b et les tiges 23c, 23d, 23e, 23f du bras 2. L'axe d'insertion 11 suit ainsi le mouvement de la rotation de deuxième type autour d'un point, appelé centre de rotation déporté 7, et situé sur l'axe (Ox) à l'intersection de l'axe d'insertion 11. L'inclinaison du bras 2 et de l'axe d'insertion 11 ainsi obtenue est observable en Fig. 4 par comparaison à la position du bras 2 en Fig. 3. Lorsque le bras est incliné, les liaisons pivot 24a à 24f permettent des rotations d'axes parallèles à un axe (Oy'), l'axe (Oy') étant l'image de l'axe (Oy) par la rotation de premier type, comme présenté en Fig. 3.

Lorsque la rotation de deuxième type, d'axe (Oy), est actionnée par le mécanisme d'actionnement, le mouvement de rotation est transmis à la tige avant 23f. La tige avant 23f effectue alors une rotation autour d'un axe parallèle à (Oy) centrée sur le centre de rotation déporté 7. L'inclinaison de l'axe d'insertion 11 ainsi obtenue est observable en Fig. 2, par comparaison à la position de l'axe d'insertion 11 en Fig. 1.

Le bras 2 du robot 1 permet de déporter les deux rotations de premier et deuxième type depuis le mécanisme d'actionnement, situé à distance d'un espace de travail du chirurgien, vers le centre de rotation déporté, situé au niveau de la zone d'intervention chirurgicale 6. L'occupation de l'espace de travail du chirurgien est ainsi limitée.

Le système d'insertion-extraction comporte un élément élastique tel que par exemple un ressort 41, un mécanisme de transmission 40a d'une rotation de troisième type et un mécanisme de transformation 40b de la rotation de troisième type en translation. Le système d'insertion-extraction comporte en outre un actionneur, tel que par exemple un-moteur rotatif permettant d'exercer un couple sur un premier élément rotatif 43 du mécanisme de transmission 40a de la rotation de troisième type. Une première extrémité du ressort 41 est reliée à une tige verticale du bras 2, telle que la tige arrière 23b ou la tige 23d, et une deuxième extrémité du ressort 41 est reliée au premier élément rotatif 43.

Le mécanisme de transmission 40a de la rotation de troisième type comporte un troisième parallélogramme déformable comportant au moins une partie de la tige supérieure 23e du double parallélogramme, une tige supplémentaire 42 parallèle à ladite tige supérieure 23e, le premier élément rotatif 43 et un deuxième élément rotatif 44. Les premier et deuxième éléments rotatifs 43 et 44 présentent chacun au moins deux zones d'assemblage leur permettant d'être reliés aux tiges 23e et 42. Les zones d'assemblage sont espacées de la même distance pour chaque élément rotatif 43, 44 et sont par exemple des cavités cylindriques d'axes parallèles. Plus précisément, la première zone d'assemblage du premier élément rotatif 43 est reliée à un premier point de la tige supérieure 23e par une liaison pivot 47c, d'axe parallèle à (Oy'), permettant au premier élément rotatif 43 de tourner par rapport à la tige supérieure 23e dans un mouvement de rotation de troisième type. De manière similaire, la première zone d'assemblage du deuxième élément rotatif 44 est reliée à un deuxième point de la tige supérieure 23e grâce à une liaison pivot 47d, d'axe parallèle à (Oy'). La tige supplémentaire 42 est reliée en une première extrémité à la deuxième zone d'assemblage du premier élément rotatif 43 par une liaison pivot 47b, et en une deuxième extrémité à la deuxième zone d'assemblage du deuxième élément rotatif 44 par une liaison pivot 47a. Les liaisons pivot 47a et 47b permettent des rotations autour d'axes parallèles à (Oy'). Ainsi, un mouvement de rotation de troisième type de l'élément rotatif 43 par rapport à la tige supérieure 23e, généré au niveau de la liaison pivot 47c par exemple par un moteur rotatif, est transmis à l'élément rotatif 44 au niveau de la liaison pivot 47d. L'élément rotatif 44 suit alors un mouvement de rotation parallèle et de même amplitude que l'élément rotatif 43 et l'actionneur est déporté vers la base 3, laissant un plus grand espace de travail au-dessus de la zone d'intervention chirurgicale 6.

Selon un mode de réalisation particulier non illustré ici, le mécanisme de transmission 40a de la rotation de troisième type comporte une courroie reliant le premier élément rotatif 43 et le deuxième élément rotatif 44 autour des axes correspondants aux liaisons pivot 47c et 47d. Ladite courroie peut ainsi transmettre un mouvement de rotation de troisième type sans nécessiter de tige supplémentaire 42.

Par ailleurs, la première extrémité du ressort 41 est reliée à une troisième zone d'assemblage de l'élément rotatif 43 positionnée de telle sorte que, lorsque l'élément rotatif tourne dans un sens anti-horaire par rapport à la tige supérieure 23e, le ressort 41 est étiré et exerce une force de traction entre l'élément rotatif 43 et une attache fixée à la tige arrière 23b, la force de traction tendant à faire entrer en rotation l'élément rotatif 43 dans un sens horaire. Selon un mode de réalisation préférentiel, la première extrémité du ressort 41 est reliée à la tige arrière 23b et la première zone d'assemblage du premier élément rotatif 43 est reliée à l'extrémité arrière de la tige supérieure 23e. Ainsi, la masse du système d'insertion-extraction et l'actionneur sont principalement déportés près de la base 3 et le plus éloignés possible de la zone d'intervention chirurgicale 6.

Selon un mode de réalisation particulier, le mécanisme de transformation 40b de la rotation de troisième type en translation comporte une bielle 45. La tête de la bielle 45 est reliée à une troisième zone d'assemblage de l'élément rotatif 44 par une liaison pivot 48a. Le pied de la bielle 45 est relié à la tige avant 23f par une liaison pivot 48b d'axe parallèle à (Oy') associée à une liaison glissière 46 d'axe de guidage parallèle à l'axe d'insertion 11. La liaison glissière 46 peut être matérialisée par une fente creusée dans la tige avant 23f. Le mouvement de rotation de troisième type de l'élément rotatif 44 est ainsi transformé en un mouvement de translation parallèle à l'axe d'insertion 11. Ledit mouvement de translation est transmis à l'outil 5, qui est relié à la fois à la tige avant 23f par la liaison glissière 46 et au pied de la bielle 45. L'outil 5 peut ainsi être relevé ou abaissé par translation selon l'axe d'insertion 11. Lors de l'insertion de l'outil 5 dans la zone chirurgicale 6, un actionneur, tel qu'un moteur rotatif, applique un couple sur l'élément rotatif 43 autour de l'axe de rotation de la liaison pivot 47c dans un sens anti-horaire. La rotation de troisième type de l'élément rotatif 43 entraîne d'une part l'élongation du ressort 41 en traction. D'autre part, la rotation de troisième type de l'élément rotatif 43 est transmise à l'élément rotatif 44 autour de l'axe de la liaison pivot 47 et transformée, par la bielle 45 reliée à la tige avant 23f, en translation vers le bas selon un axe parallèle à l'axe d'insertion 11. L'outil 5 est ainsi abaissé dans la zone d'intervention chirurgicale 6 tout en suivant l'axe d'insertion 11. Le couple appliqué par l'actionneur est maintenu pendant toute la durée d'utilisation de l'outil 5 de sorte que l'outil 5 reste dans une position abaissée.

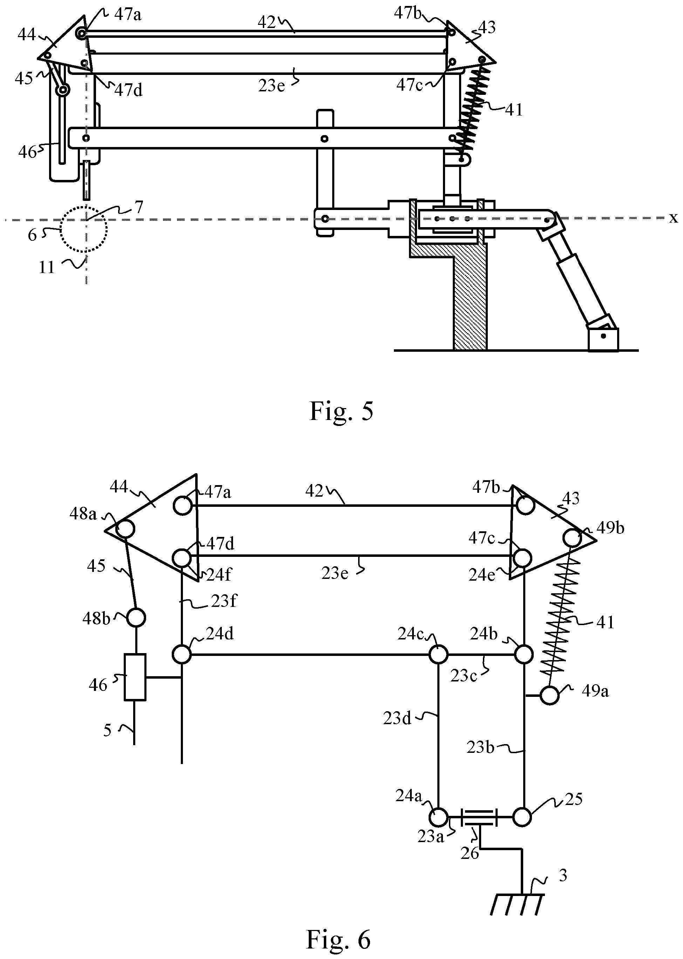

Lorsqu'un couple est appliqué par l'actionneur sur l'élément rotatif 43 en sens horaire, le mécanisme du système d'insertion-extraction entraîne inversement l'élévation de l'outil 5 et la contraction du ressort 41. La position du système d'insertion-extraction et la position relevée de l'outil 5 alors obtenues sont présentées en Fig. 5.

Lorsque le couple appliqué par l'actionneur est nul et que l'outil 5 est dans une position abaissée, la force de traction exercée par le ressort 41 sur l'élément rotatif 43 entraîne la rotation de l'élément rotatif 43 autour de l'axe de rotation de la liaison pivot 47c dans un sens horaire jusqu'à ce que le ressort 41 revienne à sa forme initiale. Ladite rotation en sens horaire est transmise à l'élément rotatif 44 puis transformée en mouvement de translation, dirigé vers le haut, permettant de relever l'outil 5 suivant l'axe d'insertion 11. L'outil 5 peut ainsi être extrait à tout moment de la zone d'intervention chirurgicale 6 avec un temps de réponse le plus rapide possible, lié uniquement au temps de réaction du ressort 41. L'extraction de l'outil 5 est en outre sécurisée et intervient de manière automatique dès que l'actionneur générant le couple appliqué à l'élément rotatif 43 est arrêté, par exemple lors d'une coupure de courant. Un bouton d'arrêt d'urgence BT, tel qu'un bouton coup de poing, peut être actionné par un individu et permet de déclencher mécaniquement une commande d'extraction qui coupe l'actionneur générant le couple appliqué à l'élément rotatif 43. Par l'effet du ressort 41, la commande d'extraction entraîne l'élévation de l'outil 5 de manière automatique. Il est ainsi possible d'extraire l'outil 5 de la zone d'intervention chirurgicale de manière sécurisée et rapide lors d'un incident inattendu. En particulier, le bouton d'arrêt d'urgence peut être utilisé lorsqu'un patient se réveille en cours d'intervention ou lors d'une coupure de courant. Dans un mode de réalisation particulier, la commande d'extraction est reliée à un système de surveillance du patient et est déclenchée lorsque le système de surveillance détecte que le patient se réveille.

Selon un mode de réalisation particulier, l'élément élastique du système d'insertion-extraction est un ressort de torsion.

L'assemblage entre l'outil 5 et la tige avant 23f n'autorisant pas de mouvement de rotation autour des axes (Ox) et (Oy), l'outil 5 reste orienté selon l'axe d'insertion 11 dans la direction de la tige avant 23f à tout moment et quelle que soit la position du système d'insertion-extraction. Ainsi, l'outil 5 est facilement orientable autour du centre de rotation déporté 7 pendant une intervention chirurgicale et il est possible d'extraire l'outil 5 de la zone d'intervention chirurgicale 6 de manière fiable à tout moment, sans risquer d'endommager un espace de passage étroit, tel qu'un conduit auditif. Il est de plus possible d'extraire puis de réinsérer l'outil 5 dans la zone d'intervention chirurgicale 6 tout en conservant la position et l'orientation de l'outil 5. Par exemple, l'outil 5 peut être extrait de la zone d'intervention chirurgicale pour effectuer une opération de nettoyage, puis réinséré de manière précise et automatique dans la zone d'intervention chirurgicale 6, à la même position et avec la même orientation qu'avant l'extraction.

L'outil 5 est préférentiellement un outil d'aide à la chirurgie qui n'entre pas en contact avec le corps humain. Ainsi, les forces et couples transmis par le système d'insertion-extraction restent faibles et dépendent principalement de la raideur du ressort 41.

Selon un mode de réalisation particulier, non illustré ici, la base 3 est mobile ou fixée à un dispositif mobile permettant trois translations : une translation selon l'axe (Ox), une translation selon l'axe (Oy) et une translation d'axe (Oz). Ainsi, le robot 1 peut être déplacé sur une table d'opération préalablement à une intervention chirurgicale de sorte que l'outil soit positionné au-dessus de la zone d'intervention chirurgicale 6.

Selon un mode de réalisation particulier, non illustré ici, le robot 1 comporte en outre un mécanisme de rotation de l'outil 5 autour de l'axe d'insertion 11. Ledit mécanisme de rotation comporte une première roue dentée d'axe parallèle à (Oy') assemblée par un engrenage à une deuxième roue dentée tournant autour de l'axe d'insertion 11. La première roue dentée est reliée à la tige avant 23f et à l'élément rotatif 44, par une liaison pivot, coaxiale aux liaisons pivot 24f et 47d. La deuxième roue dentée est fixée sur un porte-outil, relié à la tige avant 23f par la liaison glissière 46 et au pied de la bielle 45 par la liaison pivot 48b. L'outil 5 est fixé au porte-outil. Lorsque la première roue dentée entre en rotation autour d'un axe parallèle à (Oy'), elle entraîne un mouvement de rotation du porte-outil et donc de l'outil 5 autour de l'axe d'insertion 11. Ainsi, il est possible d'orienter l'outil 5 par une rotation autour de l'axe d'insertion 11 indépendamment des mouvements de rotations de premier et deuxième type et indépendamment de la position basse ou relevée de l'outil 5. Cela permet par exemple d'observer la zone d'intervention chirurgicale 6 sous différents angles dans le cas où l'outil 5 est un endoscope dont le champ de vue n'est pas centré sur 0°. Le mouvement de rotation de la première roue dentée est préférentiellement actionné de manière déportée. Par exemple, l'utilisation d'une courroie permet de relier la première roue dentée à un actionneur additionnel, tel qu'un moteur rotatif, lui-même relié à la tige arrière 23b et à l'élément rotatif 43 par une liaison pivot coaxiale aux liaisons pivot 24e et 47c. La **Fig. 2** illustre schématiquement une vue de côté du robot 1 dans une position où l'axe d'insertion 11 présente une inclinaison liée à la rotation de premier type d'axe (Oy). Les deux moteurs linéaires 31 actionnent la rotation de premier type de la tige arrière 23b par rapport à la base 3 du robot 1. L'inclinaison obtenue est transmise à la tige avant 23f et à l'axe d'insertion 11 de l'outil 5, le centre de rotation initial O étant déporté au centre de rotation déporté 7 situé à l'intersection de l'axe (Ox) et de l'axe d'insertion 11.

La **Fig. 3** illustre schématiquement le robot 1 destiné à aider la chirurgie en vue de derrière. Les deux moteurs linéaires 31a et 31b relient la base 3 à la tige arrière 23b du bras 2 par une liaison permettant d'actionner les rotations de premier et deuxième type, respectivement d'axe (Ox) et d'axe (Oy). L'axe (Ox) est perpendiculaire au plan (Ozy), autrement dit au plan de la feuille.

La tige arrière 23b du parallélogramme est reliée à la tige intermédiaire horizontale 23c et à la tige supérieure 23e par les liaisons pivot parallèles à l'axe (Oy) respectives 24b et 24e. La première zone d'assemblage de l'élément rotatif 43 est reliée à la tige supérieure 23e, la deuxième zone d'assemblage de l'élément rotatif 43 est reliée à la tige supplémentaire 42 et la troisième zone d'assemblage de l'élément rotatif 43 est rattachée à la deuxième extrémité du ressort 41. La première extrémité du ressort 41 est rattachée à la tige arrière 23b.

La **Fig. 4** illustre schématiquement en vue de derrière le robot 1 dans une position où l'axe d'insertion 11 présente une inclinaison liée à la rotation de deuxième type d'axe (Ox). Les deux moteurs linéaires 31 actionnent la rotation de deuxième type de la tige arrière 23b par rapport à la base 3 du robot 1. L'inclinaison de la tige arrière 23b ainsi obtenue est transmise à l'ensemble du bras 2 et à l'axe d'insertion 11. L'axe (Oy') est l'image de l'axe (Oy) par la rotation d'axe (Ox) effectuée.

La **Fig. 5** illustre schématiquement le robot 1 en vue de côté, le système d'insertion-extraction étant présenté dans une position relevée où l'outil 5 est hors de la zone d'intervention chirurgicale 6. Le ressort 41 n'est pas déformé.

La **Fig. 6** illustre un schéma cinématique du robot 1 dans le plan (Oxz). La base 3 est reliée à la tige inférieure 23a du bras 2 par une liaison pivot 26 d'axe (Ox). Les tiges 23a, 23b, 23c, 23d, 23e et 23f du bras 2 forment un double parallélogramme et sont reliées entre elles par les liaisons pivot 24a, 24b, 24c, 24d, 24e et 24f d'axe (Oy). Le système d'insertion-extraction est relié au bras 2 par la tige supérieure 23e et les deux liaisons pivot d'axe (Oy) 47c et 47d, qui sont, dans un exemple de réalisation, coaxiales avec les liaisons pivot respectives 24e et 24f. La tige supérieure 23e forme avec la tige 42 et les éléments rotatifs 43 et 44 un troisième parallélogramme qui s'articule autour des liaisons pivot 47a, 47b, 47c et 47d. Le troisième parallélogramme permet ainsi de transmettre une rotation de troisième type actionnée au niveau de l'élément rotatif 43 autour de l'axe de la liaison pivot 47c à l'élément rotatif 44 autour de l'axe de la liaison pivot 47d. Ladite rotation de troisième type transmise à l'élément rotatif 44 est transformée en un mouvement de translation par l'intermédiaire d'une bielle 45 reliée d'une part à l'élément rotatif 44 par une liaison pivot 48a et d'autre part à la tige avant 23f par une liaison pivot 48b et une liaison glissière 46. L'outil 5 est relié à la bielle 45 par la liaison glissière 46 et suit ledit mouvement de translation.

Le ressort 41 est relié au bras 2 par une liaison pivot 49a et à l'élément rotatif 43 du système d'insertion-extraction par une liaison pivot 49b. Lorsque l'élément 43 tourne en sens anti-horaire autour de la liaison pivot 47c et que l'outil 5 s'abaisse, le ressort 41 se déforme en s'allongeant et exerce une traction à ses extrémités sur la tige arrière 23b d'une part et l'élément rotatif 43 d'autre part. Lorsqu'un couple appliqué à l'élément rotatif 43 dans le sens anti-horaire est supprimé, la traction du ressort 41 sur l'élément rotatif 43 tend à faire tourner l'élément rotatif 43 autour de la liaison pivot 47c dans le sens horaire. L'élément rotatif 44 tourne alors de manière similaire et parallèle à l'élément 43 autour de la liaison pivot 47d dans le sens horaire, la bielle 45 transformant le mouvement de rotation de l'élément 44 en un mouvement de translation vers le haut, ce qui résulte en une élévation de l'outil 5.

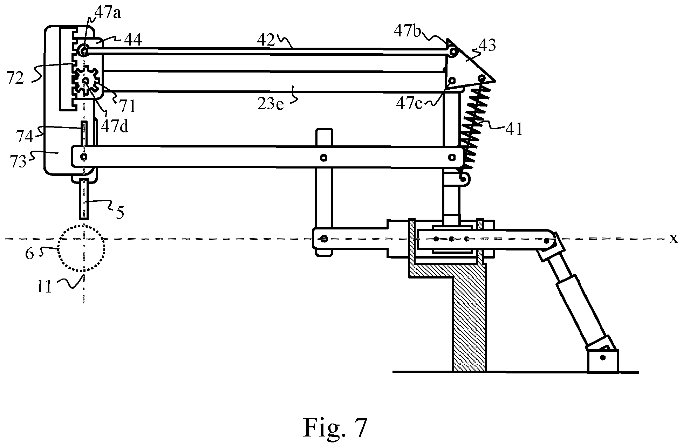

La **Fig. 7** illustre schématiquement un mode de réalisation alternatif du mécanisme de transformation 40b de la rotation de troisième type en translation. Ledit mécanisme comporte une roue dentée 71 fixée à l'élément rotatif 44 et centrée sur l'axe de la liaison pivot 47d. La roue dentée 71 est assemblée par un engrenage à une crémaillère 72, fixée sur un porte-outil 73 de telle sorte que la rotation de troisième type de la roue dentée 71 entraîne la translation du porte-outil 73 parallèlement à l'axe d'insertion 11. Le porte-outil 73 est relié à la tige avant 23f par une liaison glissière 74 permettant une translation parallèle à l'axe d'insertion 11. L'outil 5 est relié au porte-outil 73 par une liaison permettant une rotation centrée sur l'axe d'insertion 11 et suit le mouvement de translation du porte-outil 73 parallèlement à l'axe d'insertion 11.

## Revendications

1. Dispositif d'aide à la chirurgie (1) comportant des moyens pour déporter une rotation de premier type et une rotation de deuxième type, dans lequel le dispositif comporte en outre un mécanisme de transmission (40a) d'une rotation de troisième type et un mécanisme de transformation (40b) de la rotation de troisième type en une translation, ladite translation étant autorisée selon un axe d'insertion (11) orienté dans une direction définie par les moyens pour déporter la rotation de premier type et la rotation de deuxième type, le mécanisme de transmission (40a) de la rotation de troisième type étant relié à une première extrémité d'un élément élastique (41), la deuxième extrémité de l'élément élastique (41) étant reliée aux moyens pour déporter la rotation de premier type et la rotation de deuxième type, le mécanisme de transmission (40a) de la rotation de troisième type étant relié à un moteur rotatif, la rotation du moteur rotatif dans un premier sens provoquant l'abaissement d'un outil (5) porté par le dispositif d'aide à la chirurgie et une première déformation de l'élément élastique (41), la rotation du moteur rotatif dans un deuxième sens provoquant l'élévation de l'outil (5) et une deuxième déformation de l'élément élastique (41) opposée à la première déformation, l'outil (5) restant dans une position donnée tant qu'un couple exercé par le moteur rotatif est maintenu et lorsque l'outil (5) est dans une position abaissée et que le moteur rotatif n'exerce aucun couple, l'élément élastique (41) revient à une forme initiale provoquant l'élévation de l'outil (5) et en ce que les moyens pour déporter la rotation de premier type et la rotation de deuxième type comportent deux parallélogrammes reliés entre eux et un dispositif d'actionnement de la rotation de premier type et de la rotation de deuxième type, le dispositif d'actionnement générant un mouvement de rotation de premier type, autour d'un premier axe (Oy), d'une tige (23b) d'un parallélogramme par rapport à une autre tige (23a) dudit parallélogramme, et générant un mouvement de rotation de deuxième type, autour d'un deuxième axe (Ox), des deux parallélogrammes par rapport à une base (3) du dispositif d'aide à la chirurgie (1), le premier axe (Oy) étant perpendiculaire au deuxième axe (Ox), et les mouvements de rotation de premier type et de deuxième type étant transmis par les deux parallélogrammes en un centre de rotation déporté (7).

2. Dispositif selon la revendication 1, dans lequel la première déformation est une élongation.

3. Dispositif selon la revendication 1, dans lequel le dispositif d'actionnement comporte deux autres moteurs rotatifs, chacune des rotations de premier et deuxième type étant actionnée par l'un des deux autres moteurs rotatifs.

4. Dispositif selon la revendication 1, dans lequel le dispositif d'actionnement comporte deux moteurs linéaires (31, 31a, 31b), les rotations de premier et deuxième type étant actionnées par l'action conjointe des deux moteurs linéaires (31, 31a, 31b).

5. Dispositif selon l'une des revendications 1 à 4, dans lequel le mécanisme de transmission (40a) de la rotation de troisième type comporte un troisième parallélogramme, le moteur rotatif générant un mouvement de rotation de troisième type, autour d'un troisième axe, d'un premier élément rotatif (43) par rapport aux moyens pour déporter la rotation de premier type et la rotation de deuxième type, le mouvement de rotation de troisième type étant transmis par le troisième parallélogramme à un deuxième élément rotatif (44), autour d'un quatrième axe parallèle au troisième axe.

6. Dispositif selon l'une des revendications 3 à 4, dans lequel le mécanisme de transmission (40a) de la rotation de troisième type comporte une courroie, le moteur rotatif générant un mouvement de rotation de troisième type, autour d'un troisième axe, d'un premier élément rotatif par rapport aux moyens pour déporter la rotation de premier type et la rotation de deuxième type, le mouvement de rotation de troisième type étant transmis par la courroie à un deuxième élément rotatif, autour d'un quatrième axe parallèle au troisième axe.

7. Dispositif selon l'une des revendications 1 à 6, dans lequel le mécanisme de transformation (40b) de la rotation de troisième type en une translation comporte une bielle (45), la tête de la bielle (45) étant reliée au mécanisme de transmission (40a) de la rotation de troisième type et le pied de la bielle (45) étant relié à l'outil (5) et aux moyens pour déporter la rotation de premier type et la rotation de deuxième type, et lorsque le mouvement de rotation de troisième type est transmis à la tête de la bielle (45), le pied de la bielle transmet à l'outil un mouvement de translation selon l'axe d'insertion (11).

8. Dispositif selon l'une des revendications 1 à 6, dans lequel le mécanisme de transformation (40b) de la rotation de troisième type en une translation comporte une roue dentée (71) reliée au mécanisme de transmission (40a) de la rotation de troisième type et comporte une crémaillère (72) fixée sur un porte-outil (73) auquel est relié l'outil, la roue dentée (71) étant reliée à la crémaillère (72) par un engrenage de sorte que lorsque la roue dentée (71) entre en mouvement selon la rotation de troisième type, l'outil (5) effectue un mouvement de translation selon l'axe d'insertion (11).

9. Dispositif selon l'une des revendications 1 à 8, dans lequel les moyens pour déporter la rotation de premier type et la rotation de deuxième type sont reliés à une base (3) mobile, la base (3) mobile pouvant être déplacée sur une table d'opération selon trois axes de translation perpendiculaires entre eux.

10. Dispositif selon l'une des revendications 1 à 9 comportant en outre un mécanisme de rotation de l'outil autour de l'axe d'insertion (11).

11. Dispositif selon l'une quelconque des revendications 1 à 10, **caractérisé en ce que** l'outil (5) est un endoscope pour aider la chirurgie otologique.

12. Dispositif selon l'une des revendications 1 à 11 comportant en outre un moyen de commande (BT) actionné par un individu et annulant le couple exercé par le moteur rotatif de sorte que si l'outil (5) est dans une position abaissée, l'élément élastique (41) revient à la forme initiale provoquant l'élévation de l'outil (5).

## Patentansprüche

1. Chirurgisches Hilfsgerät (1) mit Mitteln, um eine Rotation erster Art und einer Rotation zweiter Art zu verlagern. Das Gerät umfasst außerdem einen Übertragungsmechanismus (40a) für eine Rotation dritter Art und einen Umwandlungsmechanismus (40b) für eine Rotation dritter Art in eine Translation. Die Translation kann entlang einer Einführachse (11), die in eine bestimmt Richtung ausgerichtet ist, durch die Mittel die Rotation der ersten Art und die Rotation zweiter Art verlagern. Der Übertragungsmechanismus (40a) für die Rotation dritter Art ist mit einem ersten Ende eines elastischen Elements (41) verbunden und das zweite Ende des elastischen Elements (41) ist mit den Mitteln zum Verlagern der Rotation erster Art und der Rotation zweiter Art verbunden. Der Übertragungsmechanismus (40a) für die Rotation dritter Art ist mit einem Drehmotor verbunden. Die Rotation des Drehmotors in eine erste Richtung bewirkt dabei das Absenken eines vom chirurgischen Hilfsgerät getragenen Werkzeugs (5) und eine erste Verformung des elastischen Elements (41) und die Rotation des Drehmotors in eine zweite Richtung bewirkt das Anheben des Werkzeugs (5) und eine zweite Verformung des elastischen Elements (41), die der ersten Verformung entgegengesetzt ist. Das Werkzeug (5) verbleibt in einer gegebenen Position, solange ein von dem Drehmotor ausgeübtes Drehmoment aufrechterhalten wird, und wenn das Werkzeug (5) in einer abgesenkten Position ist und der Drehmotor kein Drehmoment ausübt, kehrt das elastische Element (41) in eine ursprüngliche Form zurück, die das Anheben des Werkzeugs (5) bewirkt. Die Mittel zum Verlagern einer Rotation erster Art und einer Rotation zweiter Art umfassen zudem zwei miteinander verbundene Parallelogramme und eine Betätigungsvorrichtung für die Rotation erster Art und die Rotation zweiter Art. Die Betätigungsvorrichtung erzeugt eine Drehbewegung der ersten Art um eine erste Achse (Oy) eines Schafts (23b) eines Parallelogramms in Bezug auf einen anderen Schaft (23a) des Parallelogramms und erzeugt eine Drehbewegung zweiter Art um eine zweite Achse (Ox) der beiden Parallelogramme in Bezug auf eine Basis (3) des chirurgischen Hilfsgeräts (1). Dabei steht die erste Achse (Oy) senkrecht zur zweiten Achse (Ox) und die Drehbewegungen der ersten Art und der zweiten Art werden durch die beiden Parallelogramme in ein versetztes Drehzentrum (7) übertragen.

2. Gerät nach Anspruch 1, bei der die erste Verformung eine Streckung ist.

3. Gerät nach Anspruch 1, bei der die Betätigungsvorrichtung zwei weitere Drehmotoren umfasst, wobei jede der Rotationen der ersten und zweiten Art durch einen der beiden anderen Drehmotoren angetrieben wird.

4. Gerät nach Anspruch 1, bei der die Betätigungsvorrichtung zwei Linearmotoren (31, 31a, 31b) umfasst, wobei die Rotationen der ersten und zweiten Art durch die gemeinsame Wirkung der beiden Linearmotoren (31, 31a, 31b) betätigt werden.

5. Gerät nach einem der Ansprüche 1 bis 4, bei der der Übertragungsmechanismus (40a) für die Rotation dritter Art ein drittes Parallelogramm umfasst. Der Drehmotor erzeugt eine Drehbewegung der dritten Art um eine dritte Achse eines ersten Drehelements (43) in Bezug auf die Mittel zum Verlagern der Rotation erster Art und der Rotation zweiter Art. Dabei wird die Drehbewegung der dritten Art durch das dritte Parallelogramm auf ein zweites Drehelement (44) übertragen, und zwar um eine vierte Achse, die parallel zur dritten Achse verläuft.

6. Gerät nach einem der Ansprüche 3 bis 4, bei der der Übertragungsmechanismus (40a) für die Rotation dritter Art einen Riemen umfasst. Der Drehmotor erzeugt eine Drehbewegung der dritten Art um eine dritte Achse eines ersten Drehelements in Bezug auf die Mittel zum Verlagern der Rotation erster Art und der Rotation zweiter Art. Dabei wird die Drehbewegung der dritten Art durch den Riemen auf ein zweites Drehelement übertragen, und zwar um eine vierte Achse, die parallel zur dritten Achse verläuft.

7. Gerät nach einem der Ansprüche 1 bis 6, bei der der Umwandlungsmechanismus (40b) für die Rotation dritter Art in eine Translation eine Pleuelstange (45) umfasst. Der Kopf der Pleuelstange (45) ist mit dem Übertragungsmechanismus (40a) für die Rotation dritter Art und der Fuß der Pleuelstange (45) mit dem Werkzeug (5) und den Mitteln zum Verschieben zum Verlagern der Rotation erster Art und der Rotation zweiter Art verbunden. Und wenn die Drehbewegung dritter Art auf den Kopf der Pleuelstange (45) übertragen wird, überträgt der Fuß der Pleuelstange eine Translationsbewegung entlang der Einführachse (11) auf das Werkzeug.

8. Gerät nach einem der Ansprüche 1 bis 6, bei der der Umwandlungsmechanismus (40b) für die Rotation dritter Art in eine Translation ein Zahnrad (71) umfasst, das mit dem Übertragungsmechanismus (40a) für die Rotation dritter Art verbunden ist. Zudem umfasst er eine Zahnstange (72), die an einem Werkzeughalter (73) befestigt ist, mit dem das Werkzeug verbunden ist, wobei das Zahnrad (71) mit der Zahnstange (72) über ein Zahnradgetriebe verbunden ist, so dass, wenn das Zahnrad (71) gemäß der Rotation dritter Art in Bewegung kommt, das Werkzeug (5) eine Translationsbewegung gemäß der Einführachse (11) ausführt.

9. Gerät nach einem der Ansprüche 1 bis 8, bei der die Mittel zum Verlagern der Rotation erster Art und der Rotation zweiter Art mit einer beweglichen Basis (3) verbunden sind, wobei die bewegliche Basis (3) auf einem Operationstisch entlang dreier zueinander senkrechter Translationsachsen bewegt werden kann.

10. Gerät nach einem der Ansprüche 1 bis 9, die außerdem einen Rotationsmechanismus des Werkzeugs um die Einführachse (11) umfasst.

11. Gerät nach einem der Ansprüche 1 bis 10, **dadurch gekennzeichnet, dass** das Werkzeug (5) ein Endoskop zur Unterstützung der otologischen Chirurgie ist.

12. Gerät nach einem der Ansprüche 1 bis 11, die außerdem ein Steuermittel (BT) umfasst, das von einer Person betätigt wird und das vom Drehmotor ausgeübte Drehmoment aufhebt, so dass, wenn sich das Werkzeug (5) in einer abgesenkten Position befindet, das elastische Element (41) in die ursprüngliche Form zurückkehrt und somit das Anheben des Werkzeugs (5) bewirkt.

## Claims

1. Surgery-assistance device (1) comprising means for offsetting a rotation of a first type and a rotation of a second type, in which the device furthermore comprises a mechanism (40a) for transmitting a rotation of a third type and a mechanism (40b) for transforming the rotation of a third type into a translation, said translation being enabled along an insertion axis (11) oriented in a direction defined by the means for offsetting the rotation of a first type and the rotation of a second type, the mechanism (40a) for transmitting the rotation of a third type being connected to a first end of an elastic element (41), the second end of the elastic element (41) being connected to the means for offsetting the rotation of a first type and the rotation of a second type, the mechanism (40a) for transmitting the rotation of a third type being connected to a rotary motor, the rotation of the rotary motor in a first direction causing the lowering of a tool (5) carried by the surgery-assistance device and a first deformation of the elastic element (41), the rotation of the rotary motor in a second direction causing the raising of the tool (5) and a second deformation of the elastic element (41) opposite to the first deformation, the tool (5) remaining in a given position as long as a torque exerted by the rotary motor is maintained and, when the tool (5) is in a lowered position and the rotary motor is not exerting any torque, the elastic element (41) returns to an initial shape causing the raising of the tool (5), and in that the means for offsetting the rotation of a first type and the rotation of a second type comprise two parallelograms connected together and a device for actuating the rotation of a third type and the rotation of a second type, the actuation device generating a rotation movement of a first type, about a first axis (Oy), of a rod (23b) of a parallelogram with respect to another rod (23a) of said parallelogram, and generating a rotation movement of a second type, about a second axis (Ox), of the two parallelograms with respect to a base (3) of the surgery-assistance device (1), the first axis (Oy) being perpendicular to the second axis (Ox), and the rotation movements of a first type and of a second type being transmitted by the two parallelograms at an offset centre of rotation (7).

2. Device according to claim 1, wherein the first deformation is an elongation.

3. Device according to claim 1, wherein the actuation device comprises two other rotary motors, each of the rotations of a first and second type being actuated by one of the other two rotary motors.

4. Device according to claim 1, wherein the actuation device comprises two linear motors (31, 31a, 31b), the rotations of a first and second type being actuated by the conjoint action of the two linear motors (31, 31a, 31b).

5. Device according to one of claims 1 to 4, wherein the mechanism (40a) for transmitting the rotation of a third type comprises a third parallelogram, the rotary motor generating a rotation movement of a third type, about a third axis, of a first rotary element (43) with respect to the means for offsetting the rotation of a first type and the rotation of a second type, the rotation movement of a third type being transmitted by the third parallelogram to a second rotary element (44), about a fourth axis parallel to the third axis.

6. Device according to one of claims 3 to 4, wherein the mechanism (40a) for transmitting the rotation of a third type comprises a belt, the rotary motor generating a rotation movement of a third type, about a third axis, of a first rotary element with respect to the means for offsetting the rotation of a third type and the rotation of a second type, the rotation movement of a third type being transmitted by the belt to a second rotary element, about a fourth axis parallel to the third axis.

7. Device according to one of claims 1 to 6, wherein the mechanism (40b) for transforming the rotation of a third type into a translation comprises a connecting rod (45), the head of the connecting rod (45) being connected to the mechanism (40a) for transmitting the rotation of a third type and the foot of the connecting rod (45) is connected to the tool (5) and to the means for offsetting the rotation of a first type and the rotation of a second type, and, when the rotation movement of a third type is transmitted to the head of the connecting rod (45), the foot of the connecting rod transmits to the tool a translation movement along the insertion axis (11).

8. Device according to one of claims 1 to 6, wherein the mechanism (40b) for transforming the rotation of a third type into a translation comprises a toothed wheel (71) connected to the mechanism (40a) for transmitting the rotation of a third type and comprises a rack (72) secured to a tool holder (73) to which the tool is connected, the toothed wheel (71) being connected to the rack (72) by gearing so that, when the toothed wheel (71) begins to move in the rotation of a third type, the tool (5) makes a translation movement along the insertion axis (11).

9. Device according to one of claims 1 to 8, wherein the means for offsetting the rotation of a first type and the rotation of a second type are connected to a movable base (3), the movable base (3) being able to be moved and secured to an operating table in accordance with three translation axes perpendicular to each other.

10. Device according to one of claims 1 to 9, furthermore comprising a mechanism for rotating the tool about the insertion axis (11).

11. Device according to any one of claims 1 to 10, **characterised in that** the tool (5) is an endoscope for assisting ear and sinus surgery.

12. Device according to one of claims 1 to 11, furthermore comprising a control means (BT) actuated by an individual and cancelling out the torque exerted by the rotary motor so that, if the tool (5) is in a lowered position, the elastic element (41) returns to the initial shape causing the raising of the tool (5).
